# FASCICULE DE BREVET EUROPEEN

(11) **EP 2 979 091 B1**
(45) Date de publication et mention de la délivrance du brevet: **25.04.2018**
(21) Numéro de dépôt: 14714218.6
(22) Date de dépôt: 25.03.2014
(51) Int. Cl.: G01N 33/50

(54) **DOSAGE BIOLOGIQUE DES PEPTIDOGLYCANES**
BIOLOGISCHER TEST FÜR PEPTIDOGLYCAN
BIOLOGICAL ASSAY OF PEPTIDOGLYCANS

(30) Priorité: 26.03.2013 FR 1352732; 22.07.2013 FR 1357197
(43) Date de publication de la demande: 03.02.2016
(73) Titulaire: Roquette Frères, 62136 Lestrem (FR)
(72) Inventeur: LANOS, Pierre, 59480 La Bassee (FR); BIGUET, Marc, 62840 Neuve Chapelle (FR); BERNARD, Roselyne, 62136 Lestrem (FR); ALLAIN, Fabrice, 59800 Lille (FR); CARPENTIER, Mathieu, 59350 Saint Andre Lez Lille (FR); DENYS, Agnès, 59800 Lille (FR); HACINE-GHERBI, Héla, 59650 Villeneuve d'Ascq (FR)
(74) Mandataire: Gallois, Valérie
(86) Numéro de dépôt international: PCT/EP2014/055888
(87) Numéro de publication internationale: WO 2014/154651

(56) Documents cités:
- WO-A1-2012/143647
- WO-A1-2013/178931
- WO-A2-2011/158016
- ERRIDGE CLETT: "The capacity of foodstuffs to induce innate immune activation of human monocytes in vitro is dependent on food content of stimulants of Toll-like receptors 2 and 4", BRITISH JOURNAL OF NUTRITION, CAMBRIDGE UNIV. PRESS, UK, vol. 105, no. 1, 1 janvier 2011 (2011-01-01), pages 15-23, XP002690888, ISSN: 0007-1145, DOI: 10.1017/S0007114510003004 [extrait le 2010-09-20]
- R. DZIARSKI ET AL: "Staphylococcus aureus Peptidoglycan Is a Toll-Like Receptor 2 Activator: a Reevaluation", INFECTION AND IMMUNITY, vol. 73, no. 8, 22 juillet 2005 (2005-07-22), pages 5212-5216, XP055123546, ISSN: 0019-9567, DOI: 10.1128/IAI.73.8.5212-5216.2005
- C P Timmerman ET AL: "Induction of release of tumor necrosis factor from human monocytes by staphylococci and staphylococcal peptidoglycans", Infection and immunity, vol. 61, no. 10 1 octobre 1993 (1993-10-01), pages 4167-4172, XP055123665, UNITED STATES Extrait de l'Internet: URL:http://www.ncbi.nlm.nih.gov/pmc/articl es/PMC281140/pdf/iai00022-0155.pdf [extrait le 2014-06-17]
- Anonymous: "WHO | WHO International biological reference preparations", , 12 February 2013 (2013-02-12), XP055308533, Retrieved from the Internet: URL:https://web.archive.org/web/2013021208 4601/http://www.who.int/biologicals/refere nce_preparations/en/ [retrieved on 2016-10-07]

## Description

### DOMAINE DE L'INVENTION

La présente invention est relative un dosage de peptidoglycanes dans un échantillon, en particulier un échantillon de polymères de glucose.

### CONTEXTE DE L'INVENTION

Les épisodes inflammatoires aseptiques sont des complications majeures observées lors de traitements utilisant des produits manufacturés à des fins thérapeutiques (par exemple : dialyse péritonéale, alimentation parentérale, injection par voie veineuse). Si une partie de ces épisodes inflammatoires est liée à un problème d'ordre chimique (présence accidentelle de contaminants chimiques ou mauvais dosages de certains composés), la majorité des cas est consécutive à la présence de contaminants d'origine microbienne libérés au cours des processus de fabrication. Il est maintenant clairement établi que les lipopolysaccharides (LPS) et les peptidoglycanes (PGN) sont les principaux contaminants présentant un risque élevé de déclencher de tels épisodes inflammatoires lorsqu'ils sont présents à l'état de traces dans les produits manufacturés.

Le test LAL (*Limulus amebocyte lysate*) est utilisé en routine par de nombreux laboratoires de contrôle-qualité pour détecter et doser les contaminations en LPS. Ce test est basé sur la reconnaissance des endotoxines par un complexe senseur extrait de l'hémolymphe de Limule.

D'autres tests également basés sur la réactivité d'extraits d'hémolymphe d'invertébrés sont actuellement proposés pour la détection des PGN dans les produits à usage thérapeutique (SLP-Wako, Immunetics). Cependant, ces tests présentent le désavantage de ne pas être très spécifiques, puisqu'ils réagissent également avec d'autres molécules d'origine microbienne, telles que les β-glucanes. En outre, ces méthodes requièrent l'achat d'appareillage spécifique pour cette utilisation, ce qui augmente grandement les coûts et limite donc l'accès à ces méthodes de dosage.

Par ailleurs, LPS et PGN ont des structures variables en fonction de leur origine bactérienne, ce qui est responsable d'importantes différences de réactivité inflammatoire. C'est pourquoi il est d'ailleurs nécessaire d'exprimer les résultats des dosages en unités équivalentes de molécules standards (par exemple, LPS *d'E. coli* dans le test LAL).

De plus, ces molécules sont le plus souvent présentes sous forme de complexes macromoléculaires, ce qui affecte leur solubilité et leur potentiel inflammatoire. Par exemple, les PGN sont très variables en taille et sont souvent agrégés avec d'autres molécules des parois bactériennes, tels que des acides lipoteichoïques et des lipopeptides.

Ainsi, des méthodes "biologiques" ont été développées pour rendre compte uniquement de la charge inflammatoire associée à ces molécules. Les cellules effectrices de la réponse inflammatoire possèdent des senseurs spécialisés dans la reconnaissance de structures moléculaires spécifiquement produites par les agents infectieux. Ces molécules, appelée PAMPs pour *pathogen-associated molecular pattern molecules*, sont essentiellement reconnues par les TLRs (acronyme anglo-saxon de *Toll Like Receptors*) et les NLRs (acronyme anglo-saxon de *Nod-like receptors*), dont la spécificité est liée à la structure moléculaire des différentes familles de molécules inflammatoires. Contrairement au LPS qui est un ligand reconnu par les récepteurs de type TLR4), le PGN est un ligand reconnu par les récepteurs de type TLR2.

Ces dernières années, des tests cellulaires *in vitro* ont été développés pour remplacer les modèles animaux de réponse inflammatoire. La plupart de ces tests sont basés sur l'incubation de cellules monocytaires en présence des produits contaminés et sur le dosage en retour de la production en cytokines inflammatoires (TNF-α, IL-1β, IL-6, IL-8, RANTES). Toutefois, les tests utilisant des cellules primaires isolées du sang sont sujets à une importante variabilité inter-individuelle des donneurs, ce qui peut être responsable de biais expérimentaux.

A l'inverse, les lignées cellulaires monocytaires donnent des réponses constantes, ce qui explique pourquoi elles sont en général préférées aux cellules primaires. Toutefois, ces lignées n'apportent pas non plus entière satisfaction. Par exemple, le choix des cytokines est souvent critiqué, car la majorité est exprimée de façon transitoire et leur concentration dans le milieu de culture ne reflète pas toujours la charge réelle en molécules inflammatoires. Etant donné que toutes les cellules monocytaires expriment la majorité des TLRs/NLRs, les tests basés sur leur utilisation ne sont pas sélectifs d'un type de contaminant, mais vont donner une réponse inflammatoire globale.

Par ailleurs, le problème majeur vient des différences de sensibilité des cellules vis-à-vis des différentes molécules inflammatoires. Ainsi, les PGN, ligands de TLR2, sont nettement moins réactifs que les LPS, ce qui les rend difficilement détectables par ces approches. En effet, les LPS induisent une réponse significative pour des concentrations de l'ordre du ng/mL, alors que des concentrations 100 fois plus élevées en PGN sont nécessaires pour obtenir une réponse similaire (w/w ratio).

Depuis quelques années, des lignées cellulaires transfectées ont été proposées pour remplacer les modèles précédents dans les tests biologiques de détection et de quantification de la réactivité des composés inflammatoires. Ces lignées non inflammatoires (par exemple : HEK-293) sont transfectées stablement par un gène codant un récepteur spécifique d'une famille d'agonistes inflammatoires. Elles contiennent également un vecteur d'expression pour un gène rapporteur codant pour une enzyme (par exemple, la luciférase ou la phosphatase alcaline), dont la synthèse est dépendante de l'activation du récepteur inflammatoire. Ainsi, la reconnaissance d'un contaminant par les cellules exprimant le bon récepteur va déclencher la synthèse de l'enzyme, dont la production sera suivie par transformation de son substrat en produit coloré ou luminescent. Ce produit étant facilement quantifiable, cette méthode permet un dosage rapide de la réponse inflammatoire associée à un type de contaminant.

Les avantages de ces modèles cellulaires sont nombreux : remplacement des dosages ELISA des cytokines par un test enzymatique, grande reproductibilité dans les dosages en raison du caractère stable des lignées, ciblage de certaines familles de molécules inflammatoires en fonction du récepteur exprimé, détection des contaminants à des seuils très bas.

Ces modèles cellulaires peuvent donc se substituer aux tests de réponse cytokinique *in vitro*, car ils permettent de cibler spécifiquement les facteurs inflammatoires agonistes d'un TLR ou d'un NLR donné, et de quantifier la réponse inflammatoire associée à cet agoniste. Par exemple, des cellules exprimant spécifiquement TLR2 et TLR4 ont déjà été utilisées pour la détection de contaminants dans des produits alimentaires (travaux de Clett Erridge du Department of Cardiovascular Sciences de Leicester - UK dans British Journal of Nutrition, vol 105 / issue 01/ Jan 2011, pp 15-23)*.*

En outre, des sociétés telles que InvivoGen commercialisent maintenant un large éventail de cellules de la lignée HEK-293 (HEK-Blue™) transfectées avec les différents récepteurs TLRs ou NRLs. Ces cellules contiennent comme rapporteur un gène codant pour une forme sécrétée de phosphatase alcaline (SEAP: *secreted embryonic alkaline phosphatase*), ce qui permet un dosage colorimétrique aisé et rapide de la réponse aux agonistes inflammatoires.

Ces cellules HEK-Blue™ ont déjà été utilisées avec succès pour détecter la présence de contaminants dans des solutions concentrées de polymère de glucose et leur effet synergique (WO2012/143647). Comme le but affiché dans cette demande est de détecter uniquement et seulement les contaminants étant dans une forme présentant un pouvoir inflammatoire, les méthodes décrites dans cette demande de brevet ne sont pas adaptées à la mesure la quantité totale de PGN contenue dans l'échantillon. En effet, les PGN solubles (MM ≈ 120 kDa) sont ceux qui induisent une réponse inflammatoire via le récepteur TLR2. Ainsi, les PGN ne présentant pas une taille appropriée pour être inflammatoire ou agrégés avec d'autres molécules ne sont pas détectés par la méthode décrite dans cette demande.

Ainsi, il existe un besoin constant de développer des méthodes alternatives de dosage de PGN total dans un échantillon, en particulier un échantillon de polymères de glucose.

### RESUME DE L'INVENTION

La présente invention est donc relative à une méthode biologique de dosage des peptidoglycanes dans un échantillon, en particulier un échantillon de polymères de glucose.

En particulier, la présente invention est relative à un procédé de dosage de peptidoglycanes (PGN) dans un échantillon de polymère de glucose, comprenant :
a) le traitement de l'échantillon de polymère de glucose par sonication, chauffage, et/ou alcalinisation ;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur, ladite courbe étalon de correspondance entre la quantité de PGN et l'intensité du signal de gène rapporteur étant soit standardisée ou calibrée avec le PAM₃Cys-Ser-(Lys)4 trihydrochloride, soit a été préparée avec le PAM₃Cys-Ser-(Lys)4 trihydrochloride.

De préférence, le traitement de l'échantillon par sonication, chauffage, et/ou alcalinisation permet de fragmenter et désagréger les PGN contenus dans l'échantillon, en particulier de manière à les rendre capable d'activer le récepteur TLR2. En particulier, le traitement de l'échantillon permet de générer des PGN présentant majoritairement une taille d'environ 120 kDa.

De préférence, le gène rapporteur est une phosphatase alcaline sécrétée. Dans un mode de réalisation préféré, la cellule est une cellule de la lignée HEK-Blue™ hTLR2.

De préférence, la courbe étalon de correspondance entre la quantité de PGN et l'intensité du signal de gène rapporteur a été préparée avec des PGN provenant d'une bactérie choisie parmi *Staphylococcus aureus, Micrococcus luteus, Bacillus subtilis* et *Alicyclobacillus acidocaldarius,* de préférence parmi *Staphylococcus aureus, Micrococcus luteus,* et *Alicyclobacillus acidocaldarius.* En particulier, le procédé peut comprendre une étape préalable de préparation de la courbe étalon en utilisant des PGN provenant d'une bactérie choisie parmi *Staphylococcus aureus, Micrococcus luteus, Bacillus subtilis* et *Alicyclobacillus acidocaldarius,* de préférence parmi *Staphylococcus aureus, Micrococcus luteus,* et *Alicyclobacillus acidocaldarius.*

De préférence, l'échantillon est dilué, si nécessaire, de manière à générer un signal du gène rapporteur correspondant à la partie linéaire de la courbe étalon.

De préférence, l'échantillon est un échantillon d'une solution d'icodextrine.

De préférence, la courbe étalon de correspondance entre la quantité de PGN et l'intensité du signal de gène rapporteur est standardisée ou calibrée avec le PAM₃Cys-Ser-(Lys)4 trihydrochloride.

Dans un mode de réalisation alternatif, la courbe étalon de correspondance entre la quantité de PGN et l'intensité du signal de gène rapporteur a été préparée avec le PAM₃Cys-Ser-(Lys)4 trihydrochloride. En particulier, le procédé peut comprendre une étape préalable de préparation de la courbe étalon en utilisant le PAM₃Cys-Ser-(Lys)4 trihydrochloride.

L'invention est en outre relative à un kit permettant le dosage de peptidoglycanes (PGN) dans un échantillon de polymères de glucose, comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ; et
- un standard de PGN, provenant de préférence d'une bactérie choisie parmi *Staphylococcus aureus, Micrococcus luteus, Bacillus subtilis* et *Alicyclobacillus acidocaldarius,* de préférence parmi *Staphylococcus aureus*, *Micrococcus luteus*, et *Alicyclobacillus acidocaldarius*,
- facultativement une notice d'utilisation et/ou une solution de pré-traitement de l'échantillon ;
ledit kit comprenant en outre le PAM₃Cys-Ser-(Lys)4 trihydrochloride.

Enfin, l'invention concerne l'utilisation d'un kit pour le dosage de peptidoglycanes (PGN) dans un échantillon de polymères de glucose selon le procédé de l'invention, ledit kit comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ; et
- soit une courbe étalon de correspondance entre la quantité de PGN et l'intensité du signal de gène rapporteur, soit un standard de PGN, provenant de préférence d'une bactérie choisie parmi *Staphylococcus aureus*, *Micrococcus luteus*, *Bacillus subtilis* et *Alicyclobacillus acidocaldarius,* de préférence parmi *Staphylococcus aureus*, *Micrococcus luteus*, et *Alicyclobacillus acidocaldarius*;
- facultativement une notice d'utilisation, une solution de pré-traitement de l'échantillon ;
ledit kit comprenant en outre le PAM₃Cys-Ser-(Lys)4 trihydrochloride.

### DESCRIPTION DETAILLEE DE L'INVENTION

La présente invention est donc relative à une méthode biologique de dosage des peptidoglycanes dans un échantillon de polymères de glucose tel que défini dans les revendications. En particulier, la présente description décrit un procédé de dosage de peptidoglycanes (PGN) dans un échantillon de polymère de glucose, comprenant :
a) le traitement de l'échantillon de polymère de glucose par sonication, chauffage, et/ou alcalinisation ;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur.

De préférence, les polymères de glucose sont destinés à la dialyse péritonéale, la nutrition entérale et parentérale et l'alimentation des nouveaux nés. Dans un aspect préféré, les polymères de glucose qui seront testés sont de l'icodextrine ou des maltodextrines. En particulier, ils peuvent être destinés à la préparation de dialyse péritonéale. Ils peuvent être testés à un ou plusieurs stades de leur préparation, et notamment au niveau de la matière première, à une étape quelconque de leur procédé de préparation, et/ou au niveau du produit final du procédé. Ils peuvent également être testés en tant qu'échantillon d'une solution de dialyse péritonéale.

Dans une première étape du procédé, l'échantillon de polymère de glucose est traité par sonication, chauffage, et/ou alcalinisation. Le but de ce traitement est de fragmenter les PGN et/ou de désagréger les PGNs contenus ou piégés dans des agrégats, le but étant de générer des PGNs capables d'interagir avec les récepteurs TLR2 et de les activer. Comme indiquer précédemment, ce traitement doit permettre de désagréger les PGNs contenus ou piégés dans des agrégats et de fragmenter les PGNs de taille trop élevés, notamment pour générer des PGNs solubles de tailles comprises entre 30 et 5000 kDa, notamment d'environ 120 kDa. Cependant, le traitement ne doit pas affecter la capacité des PGNs à interagir avec les récepteurs TLR2. Il est de préférence optimisé pour libérer au maximum de PGNs capables d'interagir avec TLR2 et d'activer le récepteur et pour conserver un maximum de PGNs déjà actifs sur TLR2.

Dans un premier mode de réalisation, le traitement de l'échantillon comprend au moins une étape de sonication. Facultativement, la sonication peut durer de 30 secondes à 5 minutes, utiliser une puissance de 20 à 40 kHz et/ou comprendre un ou plusieurs cycles de sonication, par exemple de 1 à 5 cycles. Dans un mode de réalisation préféré, l'échantillon sera traité par une sonication pendant 1 minute à 35 kHz en un cycle unique. Facultativement, le traitement par sonication peut être combiné avec un traitement par la chaleur et/ou par alcalinisation.

Dans un deuxième mode de réalisation, le traitement de l'échantillon comprend au moins une étape d'alcalinisation. De préférence, l'agent alcalinisant est NaOH, notamment à une concentration comprise entre 0,1 et 1 M. Facultativement, la durée de l'étape d'alcalinisation peut durer de 5 minutes à 60 minutes. Facultativement, l'étape d'alcalinisation peut être réalisée à une température élevée, notamment une température comprise entre 20 °C et 80 °C, par exemple à une température de 20, 40, 60 ou 80 °C. Facultativement, le traitement par alcalinisation peut être combiné avec un traitement par sonication.

Dans un deuxième mode de réalisation, le traitement de l'échantillon comprend au moins une étape de chauffage. Facultativement, la durée de l'étape de chauffage peut durer de 5 minutes à 60 minutes. Facultativement, l'étape de chauffage peut être réalisée à une température élevée, notamment une température comprise entre 20 °C et 80 °C, par exemple à une température de 20, 40, 60 ou 80 °C. Facultativement, le traitement de chauffage peut être combiné avec un traitement par sonication et/ou par alcalinisation.

Les modes de traitement de l'échantillon ne comportent pas d'étapes de traitement enzymatique, notamment par une mutanolysine.

Dans une étape subséquente, l'échantillon et/ou des dilutions de celui-ci est mis en contact avec des cellules recombinantes exprimant le récepteur TLR2. Les cellules sont qualifiées de recombinantes car ce sont des cellules qui ont été modifiées par l'introduction d'un acide nucléique codant pour la récepteur TLR2, de préférence le récepteur TLR2 humain, la cellule initiale n'exprimant pas TLR2.

L'activité du récepteur TLR2 est détectée en utilisant un gène rapporteur qui est sous la dépendance directe de la voie de signalisation associée audit récepteur. De manière préférée, ce gène rapporteur code pour une protéine colorée ou fluorescente, ou pour une protéine dont l'activité peut être mesurée avec ou sans substrat. De manière particulière, le gène rapporteur code pour une phosphatase alcaline. Notamment, le gène rapporteur peut produire une forme sécrétée de la phosphatase alcaline (acronyme anglo-saxon SEAP: *secreted embryonic alkaline phosphatase*), dont la synthèse est sous la dépendance directe de la voie de signalisation associée à TLR2.

Dans un mode de réalisation préférée, la lignée cellulaire utilisée est une lignée HEK-Blue™ (commercialisée par la société InvivoGen), modifiées par transfection stable avec des vecteurs codant pour TLR2 humain : la lignée HEK-Blue™ hTLR2. Cependant, il est à noter que l'homme du métier peut également utiliser d'autres lignées commercialement (Imgenex) ou il peut en préparer.

Lorsque la cellule est HEK-Blue™ hTLR2, la cellule est de préférence mise en oeuvre à une densité d'environ 50 000 cellules/puits pour une plaque de 96 puits.

Dans un mode de réalisation particulier, l'échantillon de polymères de glucose ou une dilution de celui-ci présente une concentration de polymères de glucose de 5 à 50 mg/mL, de préférence entre 5 et 10, 20, 30 ou 40 mg/mL. Dans le mode de réalisation préféré, la l'échantillon de polymères de glucose ou une dilution de celui-ci présente une concentration de polymères de glucose d'environ 37,5 mg/mL.

Dans un autre mode de réalisation particulier, l'échantillon de polymères de glucose ou une dilution de celui-ci présente une concentration maximale de polymère de glucose de 3,75 % (poids/volume), de préférence 3 %.

Par exemple, les échantillons sont préparés de sorte à avoir une concentration de polymère de glucose de 3,75 % (poids/volume), de préférence 3 %, et les échantillons sont soumis au procédé de dosage selon la présente invention en testant l'échantillon ainsi que des dilutions 1/10, 1/100 et 1/1000.

De préférence, la mise en contact de l'échantillon de polymères de glucose ou d'une dilution de celui-ci avec les cellules dure environ 5 à 48 h, de préférence de 10 à 36 h, de préférence encore de 16 à 24 h.

Ensuite, le procédé comprend la mesure du signal de gène rapporteur.

Dans un mode de réalisation préféré mettant en oeuvre la lignée HEK-Blue™ hTLR2, le signal est la mesure de l'activité de la phosphatase alcaline. De préférence, la réaction enzymatique est mise en oeuvre en utilisant un ratio 1 : 3 de milieu à tester versus le réactif SEAP (par exemple 50 µL de milieu et 150 µL de réactif SEAP). En outre, un temps de réaction d'au moins 60 minutes sera préféré.

Enfin, la quantité de PGN dans l'échantillon est déterminée à l'aide d'une courbe étalon de correspondance entre la quantité de PGN et l'intensité du signal de gène rapporteur.

Cette courbe est de préférence réalisée avec les mêmes cellules, dans les mêmes conditions, avec des doses croissantes de PGNs, en particulier des standards de PGN.

Le standard de PGN peut être tout PGN d'origine bactérienne. Par exemple, les PGN peuvent provenir des microorganismes suivants : *Staphylococcus aureus, Micrococcus luteus, Escherichia coli*, *Bacillus subtilis* et *Alicyclobacillus acidocaldarius.* En particulier, les standards utilisés sont des PGNs purifiés et partiellement digérés. De tels standards sont disponibles commercialement (Invitrogen, Catalog # tlrl-pgnec or tlrl-pgnek from *E coli* ; Catalog # tlrl-pgnb2 from *B subtilis* ; Catalog # tlrl-pgnsa from *S aureus*) (Wako Pure Chemical, Catalog # 162-18101 from *M luteus*).

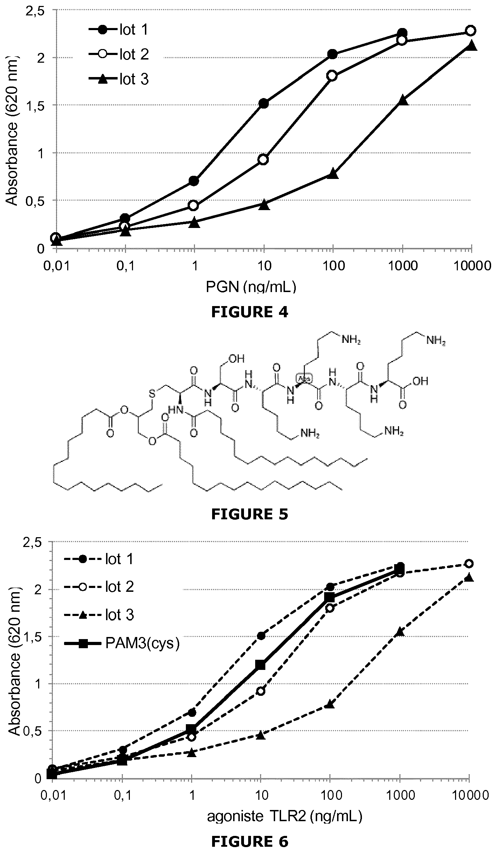

Le standard de PGN est de préférence calibré en utilisant un étalon interne agoniste de TLR2, de façon à exprimer les résultats en unités équivalentes de PGN actif. L'étalon interne peut être un lipopeptide, de préférence de synthèse, en particulier le PAM₃Cys-Ser-(Lys)4 trihydrochloride (Pam3(cys), PAM ou Pam signifiant acide palmitique) (voir Figure 5). Ainsi, la courbe étalon de correspondance entre la quantité de PGN et l'intensité du signal de gène rapporteur est de préférence standardisée ou calibrée avec un étalon interne agoniste de TLR2, de préférence un lipopeptide, en particulier le PAM₃Cys-Ser-(Lys)4 trihydrochloride. Cet étalon interne est de préférence de synthèse ou à une structure/composition bien définie. La calibration ou standardisation est réalisée en comparant les pentes des parties linéaires de chaque courbe dose-réponse et en calculant un facteur de correction permettant de superposer la courbe obtenue avec l'étalon de calibration et celle du standard PGN.

Par exemple, la courbe étalon peut être réalisée en utilisant des concentrations de PGNs allant de 0,001 à 1 000 ng/mL, notamment de 0,01 à 100 ng/mL.

Cette courbe étalon peut être réalisée soit avec des PGNs uniquement, soit avec une solution de polymère de glucose dans laquelle des quantités définies de PGNs ont été ajoutées. Notamment, la solution de polymère de glucose utilisée peut comprendre 3,75 % (poids/volume) de polymère de glucose, de préférence 3 %.

Cette courbe de correspondance entre la quantité de PGN et l'intensité du signal de gène rapporteur peut également être réalisée avec un étalon interne agoniste de TLR2, de préférence un lipopeptide, en particulier le PAM₃Cys-Ser-(Lys)4 trihydrochloride, notamment avec les mêmes cellules, dans les mêmes conditions, avec des doses croissantes d'étalon interne agoniste de TLR2. Cet étalon interne est de préférence de synthèse ou à une structure/composition bien définie. De même que pour le PGN, elle peut être réalisée en absence ou en présence de polymère de glucose, de préférence en présence.

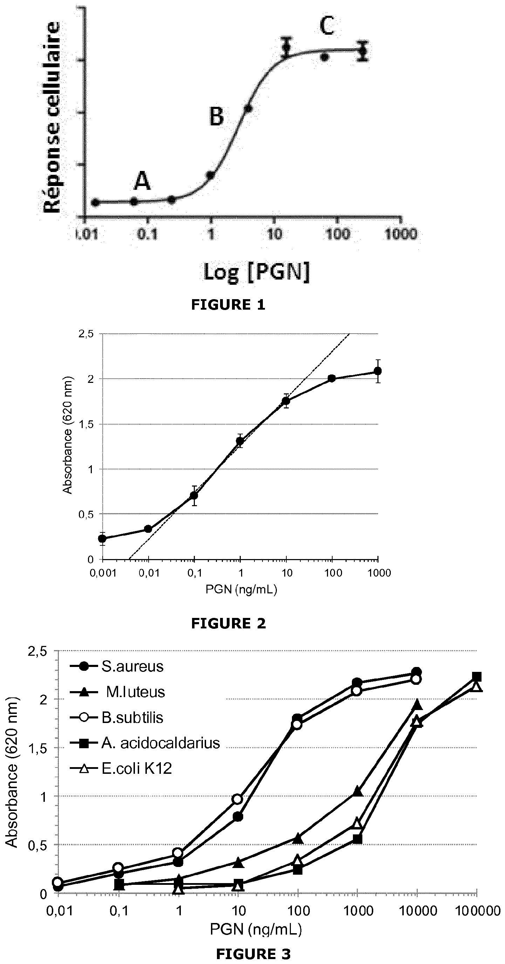

Typiquement, la courbe étalon est une courbe classique de réponse cellulaire de type sigmoïde (Figure 1).
- la partie (A) correspond aux réponses obtenues avec des concentrations faibles en PGN, en-deçà de celles donnant une activation efficace de TLR2. Cette zone non linéaire correspond donc au seuil limite de détection de la méthode. De façon à inclure la variabilité de la méthode, on estime ce seuil de détection à trois fois la valeur du bruit de fond (réponse obtenue en absence de stimulus).
- la partie (B) est la plus intéressante car on observe une réponse linéaire. Cette zone de réponse efficace permet de déterminer une relation directe entre la réponse cellulaire et le taux de PGN. Il s'agit donc de la zone de dosage.
- la partie (C) correspond à une saturation de la réponse cellulaire en présence de concentrations trop fortes en PGN. Il y a en fait une saturation des récepteurs TLR2.

On se place dans la partie linéaire de la courbe étalon, cette partie correspondant à une zone (partie B) où la quantité de PGN est directement proportionnelle au signal du gène rapporteur.

Dans le cas d'échantillons susceptibles d'être fortement contaminés en PGN, il faudra réaliser plusieurs dilutions en série de façon à toujours se localiser dans la zone de linéarité. A l'inverse, des concentrations faibles en PGN nécessitent une étape de concentration de l'échantillon si l'on veut augmenter la sensibilité du dosage.

Facultativement, le procédé comprend en outre un test avec une cellule contrôle qui n'exprime pas TLR2, plus généralement qui n'exprime pas de récepteur de l'immunité innée. Par exemple, la lignée HEK-Blue™ Null2 peut être utilisée. Il s'agit d'une lignée contrôle, dont l'utilisation est utile pour vérifier que l'échantillon de polymères de glucose n'induit pas la production de l'enzyme par un mécanisme intrinsèque.

La présente description décrit un kit permettant le dosage de peptidoglycanes (PGN) dans un échantillon de polymères de glucose, le kit comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat. Notamment, la cellule est de préférence la lignée HEK-Blue™ hTLR2. A titre de témoin négatif, le kit peut également comprendre une cellule n'exprimant pas de récepteur de l'immunité innée, par exemple la lignée HEK-Blue™ Null2.
- soit une courbe étalon de correspondance entre la quantité de PGN et l'intensité du signal de gène rapporteur, soit un standard calibré de PGN, provenant de préférence d'une bactérie choisie parmi *Staphylococcus aureus*, *Micrococcus luteus*, *Escherichia coli*, et *Alicyclobacillus acidocaldarius*, de préférence *Staphylococcus aureus,* soit un étalon interne agoniste de TLR2, de préférence un lipopeptide, en particulier le PAM₃Cys-Ser-(Lys)4 trihydrochloride. Facultativement, le kit peut comprendre les deux, c'est-à-dire une courbe étalon ainsi que un standard calibré de PGN provenant du même microorganisme que celui utilisé pour préparer cette courbe étalon.
- facultativement une notice d'utilisation, une solution de pré-traitement de l'échantillon, les réactifs utiles pour mesurer la réponse du gène rapporteur, des microplaques, etc...

De préférence, le kit comprend en outre un étalon interne agoniste de TLR2, de préférence un lipopeptide, en particulier le PAM₃Cys-Ser-(Lys)4 trihydrochloride.

### DESCRIPTION DES FIGURES

FIGURE 1 : Courbe théorique de la réponse cellulaire en fonction de concentrations croissantes de PGN.
FIGURE 2 : Courbe étalon de la réponse cellulaire en fonction du taux de PGN de *S*. *aureus* obtenue avec les cellules HEK-Blue™-hTLR2.
FIGURE 3 : Réponse des cellules HEK-Blue™-hTLR2 en fonction des concentrations croissantes en PGN de différentes espèces bactériennes..
FIGURE 4 : Réponse des cellules HEK-Blue™-hTLR2 en fonction des concentrations croissantes en PGN de *S. aureus* issu de lots différents.
FIGURE 5 : Structure du PAM₃Cys-Ser-(Lys)4 trihydrochloride (PAM3(cys)).
FIGURE 6 : Comparaison des réponses induites par les PGN de *S. aureus* et le PAM3(cys) dans les cellules HEK-Blue™-hTLR2.
FIGURE 7 : Réponse des cellules HEK-Blue™-hTLR2 en fonction des concentrations corrigées en PGN.
FIGURE 8 : Courbe étalon de la réponse des cellules HEK-Blue™-hTLR2 en fonction des concentrations corrigées en PGN actif.

### EXEMPLES

Le dosage est basé sur la reconnaissance spécifique des PGNs par une lignée exprimant le récepteur TLR2 et sur la production d'une activité enzymatique mesurable via l'activation de la voie de signalisation associée à TLR2.

### Matériel cellulaire

Pour les expériences relatives à ce dosage, deux lignées sont utilisées :
- lignée HEK-Blue™ hTLR2 (HEK-TLR2): réponse spécifique pour les ligands de TLR2, avec une forte réactivité pour les PGN solubles.
- lignée HEK-Blue™ Null2 (HEK-Null): réponse non spécifique liée à un effet cytotoxique de l'échantillon.

Les cellules sont cultivées selon les recommandations du fournisseur (InvivoGen). A 75% de confluence, les cellules sont remises en suspension à une densité de 0,28x10⁶ cellules/mL. Avant stimulation, 180 µL de la suspension cellulaire sont répartis dans les puits de culture (boite de 96 puits), soit 50 000 cellules/puits. Les cellules sont ensuite stimulées pendant 24 h par addition de 20 µL des échantillons de polymère de glucose à 37,5 % (poids/volume) (soit une dilution finale des échantillons à 3,75 %). Après 24 h de stimulation, la réponse cellulaire est mesurée par quantification de l'activité enzymatique produite.

### 1- Etablissement de la courbe doses-réponses

Une courbe doses-réponses a été réalisée en diluant différentes quantités de PGN standard de *S. aureus* (Figure 2) dans une solution d'icodextrine non contaminée préparée à 37,5 % (poids/volume) (Figure 2).

Le résultat est une courbe classique de réponse cellulaire de type sigmoïde.
- la partie (A) correspond aux réponses obtenues avec des concentrations faibles en PGN, en-deçà de celles donnant une activation efficace de TLR2. Cette zone non linéaire correspond donc au seuil limite de détection de la méthode.
- la partie (B) est la plus intéressante car on observe une réponse linéaire. Cette zone de réponse efficace permet de déterminer une relation directe entre la réponse cellulaire et le taux de PGN. Il s'agit donc de la zone de dosage.
- la partie (C) correspond à une saturation de la réponse cellulaire en présence de concentrations trop fortes en PGN. Il y a en fait une saturation des récepteurs TLR2.

La courbe standard de réponse des cellules HEK-TLR2 au PGN de *S. aureus* présente une zone de linéarité pour des concentrations comprises entre 0,07 et 10 ng/mL (soit entre 2 et 267 ng/g d'icodextrine).

### 2- Etablissement de la courbe étalon pour le dosage biologique des PGN avec un étalon interne

Les courbes dose-réponse ont été réalisées en diluant les PGN de différentes espèces bactériennes dans une solution de maltodextrine non contaminée (référencée P-11.11) préparée à 37,5% (poids/volume). Les PGN testés sont extraits de *Staphylococcus aureus* (Sigma, Cat No 77140), *Micrococcus luteus* (Sigma, Cat No 53243), *Bacillus subtilis* (InvivoGen, # tlrl-pgnb2), et *Alicyclobacillus acidocaldarius* (préparation personnelle).

Les courbes obtenues sont classiques des réponses observées dans les dosages réalisés avec un matériel cellulaire (*bio-assay*) (Figure 3). Les valeurs d'absorbance inférieures à 0,2 témoignent de concentrations trop faibles en PGN pour induire une réponse cellulaire, alors que des valeurs supérieures à 2 montrent un effet de plateau lié à la saturation des récepteurs TLR2. Par conséquent, seule la zone comprise entre ces deux valeurs d'absorbances limites permet de corréler la production de SEAP à la quantité de PGN présente dans les échantillons.

Les réponses observées montrent une large variabilité dans la réactivité cellulaire associée à chaque type de PGN. En effet, les concentrations donnant une réponse égale à 50% de la réponse maximale (EC50) sont de ∼ 20 ng/mL pour les PGN de *S. aureus* et *B. subtilis*, 1500 ng/mL pour *M. luteus*, et plus de 2000 ng/mL pour ceux extraits de *A*. *acidocaldarius* et *E.coli* K12.

Ces différences étaient toutefois attendues, puisque les PGN ont des structures différentes en fonction de leur origine bactérienne, ce qui est responsable d'importantes variations de réactivité inflammatoire. Ces observations soulignent l'importance de définir un standard interne de façon à pouvoir exprimer les résultats en unités équivalentes de PGN.

Un autre facteur susceptible de modifier la réponse des cellules HEK-TLR2 est la taille des PGN, qui va influencer leur solubilité et la réactivité vis-à-vis de TLR2. Ainsi, la procédure de purification de ces macromolécules peut largement influencer la réponse des cellules, puisque les conditions d'extraction vont pouvoir modifier la taille des PGN, voire provoquer une dégradation partielle. Pour tester cette hypothèse, les tests ont été reproduits avec 3 lots distincts de PGN extraits de *S. aureus* : 2 lots Sigma (Cat No 77140 : lot 1, 0001442777 ; lot 2, BCBH7886V) et 1 lot InvivoGen (# tlrl-pgnsa).

Les résultats montrent une variabilité de réactivité entre les trois lots (Figure 4). En effet, les EC50 sont respectivement de 4, 20 et 400 ng/mL pour les trois lots. Ces données indiquent que des PGN extraits de la même espèce bactérienne risquent de présenter des différences de réactivité, même si les lots proviennent du même fournisseur et ont été à priori extraits par la même procédure. Il apparait donc nécessaire d'introduire un étalon interne pour la courbe étalon, de façon à éviter des erreurs relatives à la variabilité des PGN et à exprimer les résultats en quantité de PGN « actif ».

Le PAM₃Cys-Ser-(Lys)4 trihydrochloride (PAM3(cys) ; Figure 5) est un lipopeptide de synthèse triacylé qui mime la structure des lipopeptides bactériens et agit comme un agoniste fort de TLR2. Etant de structure homogène, il est souvent utilisé comme témoin positif pour calibrer les réponses des cellules exprimant le récepteur TLR2.

Les expériences ont donc été reproduites en remplaçant le PGN par le PAM3(cys) dans nos essais. Comme attendu, les cellules HEK-TLR2 montrent une forte réactivité vis-à-vis de ce composé. De plus, l'allure de la courbe dose-réponse est similaire à celles obtenues en présence de PGN, avec une EC50 estimée à 10 ng/mL (Figure 6). Ces résultats indiquent que le PAM3(cys) induit des réponses équivalentes à celles des PGN les plus réactifs, mais contrairement à ces derniers, il ne présente pas de variabilité structurale. Par conséquent, ce lipopeptide de synthèse peut être utilisé pour calibrer les lots de PGN et établir une courbe étalon standardisée, ce qui permettra de formuler les résultats en quantités de PGN « actif », c'est-à dire en quantités de PGN donnant des réponses TLR2 identiques à celles obtenues avec les mêmes quantités de PAM3(cys).

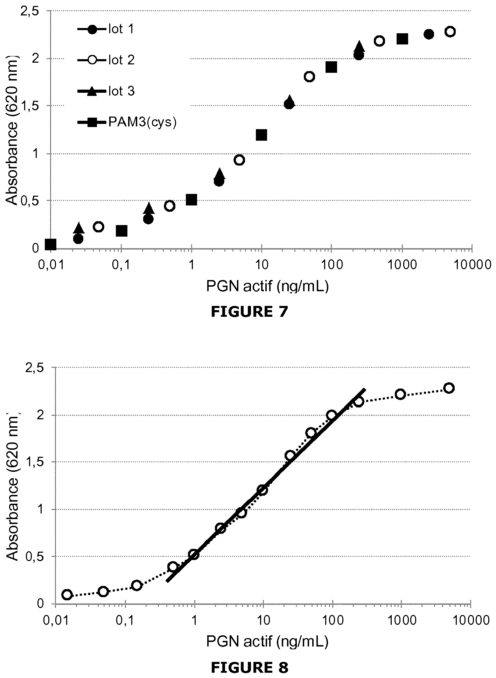

La calibration de chaque lot de PGN par rapport au PAM3(cys) est réalisée en comparant les pentes des parties linéaires de chaque courbe dose-réponse, et en calculant un facteur de correction pour superposer les courbes des PGN à celle du PAM3(cys). Dans l'exemple présenté en Figure 6, les facteurs de corrections ont été estimés à 0,4, 2 et 40 respectivement pour les lots 1, 2 et 3. Cela signifie qu'il faut 2,5 fois moins de PGN du lot 1 pour obtenir des réponses identiques à celles induites par le PAM3(cys), mais 2 fois plus de PGN du lot 2 et 40 fois plus de PGN du lot 3. Après avoir corrigé les quantités brutes de PGN, on peut voir que tous les points s'alignent sur une même courbe qui se superpose à celle obtenue avec le PAM3(cys) (Figure 7). Par conséquent, l'utilisation de l'étalon interne permet d'obtenir des concentrations corrigées pour tous les lots de PGN et d'établir une courbe dose-réponse calibrée en PGN actif.

En appliquant cette méthode, la courbe standard de réponse des cellules HEK-TLR2 présente une zone de linéarité pour des concentrations de PGN actif comprises entre 0,5 et 200 ng/mL (Figure 8), soit entre 13 et 5400 ng/g de polymères de glucose.

## Revendications

1. Procédé de dosage de peptidoglycanes (PGN) dans un échantillon de polymère de glucose, comprenant :
a) le traitement de l'échantillon de polymère de glucose par sonication, chauffage, et/ou alcalinisation;
b) la mise en contact de l'échantillon traité ou d'une dilution de celui-ci avec une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ;
c) la mesure du signal de gène rapporteur ; et
d) la détermination de la quantité de PGN dans l'échantillon à l'aide d'une courbe étalon de correspondance entre la quantité de PGN et l'intensité du signal de gène rapporteur **caractérisé en ce que** ladite courbe étalon de correspondance entre la quantité de PGN et l'intensité du signal de gène rapporteur est soit standardisée ou calibrée avec le PAM₃Cys-Ser-(Lys)4 trihydrochloride, soit a été préparée avec le PAM₃Cys-Ser-(Lys)4 trihydrochloride.

2. Procédé selon la revendication 1, dans lequel le traitement de l'échantillon par sonication, chauffage, et/ou alcalinisation permet de fragmenter et désagréger les PGN contenus dans l'échantillon, en particulier de manière à les rendre capable d'activer le récepteur TLR2.

3. Procédé selon la revendication 1 ou 2, dans lequel le traitement de l'échantillon permet de générer des PGN présentant majoritairement une taille d'environ 120 kDa.

4. Procédé selon l'une quelconque des revendications 1-3, dans lequel le gène rapporteur est une phosphatase alcaline sécrétée.

5. Procédé selon l'une quelconque des revendications 1-4, dans lequel la cellule est une cellule de la lignée HEK-Blue™ hTLR2.

6. Procédé selon l'une quelconque des revendications 1-5, dans lequel la courbe étalon de correspondance entre la quantité de PGN et l'intensité du signal de gène rapporteur est standardisée ou calibrée avec le PAM₃Cys-Ser-(Lys)4 trihydrochloride , et dans lequel la courbe étalon de correspondance entre la quantité de PGN et l'intensité du signal de gène rapporteur a été préparée avec des PGN provenant d'une bactérie choisie parmi *Staphylococcus aureus, Micrococcus luteus, Bacillus subtilis* et *Alicyclobacillus acidocaldarius,* de préférence parmi *Staphylococcus aureus, Micrococcus luteus,* et *Alicyclobacillus acidocaldarius.*

7. Procédé selon l'une quelconque des revendications 1-6, dans lequel le procédé comprend une étape préalable de préparation de la courbe étalon en utilisant des PGN provenant d'une bactérie choisie parmi *Staphylococcus aureus, Micrococcus luteus, Bacillus subtilis* et *Alicyclobacillus acidocaldarius.*

8. Procédé selon l'une quelconque des revendications 1-5 dans lequel la courbe étalon de correspondance entre la quantité de PGN et l'intensité du signal de gène rapporteur a été préparée avec le PAM₃Cys-Ser-(Lys)4 trihydrochloride, et dans lequel le procédé comprend une étape préalable de préparation de la courbe étalon en utilisant le PAM₃Cys-Ser-(Lys)4 trihydrochloride.

9. Procédé selon l'une quelconque des revendications 1-8, dans lequel l'échantillon est dilué, si nécessaire, de manière à générer un signal du gène rapporteur correspondant à la partie linéaire de la courbe étalon.

10. Procédé selon l'une quelconque des revendications 1-9, dans lequel l'échantillon est un échantillon d'une solution d'icodextrine.

11. Kit permettant le dosage de peptidoglycanes (PGN) dans un échantillon de polymères de glucose comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ; et
- un standard de PGN, provenant de préférence d'une bactérie choisie parmi *Staphylococcus aureus, Micrococcus luteus, Bacillus subtilis* et *Alicyclobacillus acidocaldarius,* de préférence parmi *Staphylococcus aureus, Micrococcus luteus,* et *Alicyclobacillus acidocaldarius;*
- facultativement une notice d'utilisation, une solution de pré-traitement de l'échantillon ;
ledit kit comprenant en outre le PAM₃Cys-Ser-(Lys)4 trihydrochloride.

12. Utilisation d'un kit pour le dosage de peptidoglycanes (PGN) dans un échantillon de polymères de glucose selon le procédé de l'une quelconque des revendications 1 à 10, ledit kit comprenant :
- une cellule recombinante exprimant un récepteur TLR2 (Toll-like Receptor 2) exogène et un gène rapporteur sous la dépendance directe de la voie de signalisation associée au récepteur TLR2, le gène rapporteur codant pour une protéine colorée ou fluorescente ou pour une protéine dont l'activité peut être mesurée avec ou sans substrat ; et
- soit une courbe étalon de correspondance entre la quantité de PGN et l'intensité du signal de gène rapporteur, soit un standard de PGN, provenant de préférence d'une bactérie choisie parmi *Staphylococcus aureus, Micrococcus luteus, Bacillus subtilis* et *Alicyclobacillus acidocaldarius,* de préférence parmi *Staphylococcus aureus, Micrococcus luteus,* et *Alicyclobacillus acidocaldarius;*
- facultativement une notice d'utilisation, une solution de pré-traitement de l'échantillon ;
ledit kit comprenant en outre le PAM₃Cys-Ser-(Lys)4 trihydrochloride.

## Patentansprüche

1. Verfahren zur quantitativen Bestimmung von Peptidoglycanen (PGN) in einer Probe von Glucosepolymer, umfassend
a) Behandlung der Probe von Glucosepolymer mittels Beschallung, Erwärmung und/oder Alkalisierung;
b) Inkontaktbringen der behandelten Probe oder einer Verdünnung davon mit einer rekombinanten Zelle, die einen exogenen TLR2-Rezeptor (Toll-like Receptor 2) und ein Reportergen in direkter Abhängigkeit des TLR2-Rezeptor-assoziierten Signalwegs exprimiert, wobei das Reportergen für ein farbiges oder fluoreszierendes Protein oder für ein Protein codiert, dessen Aktivität mit oder ohne Substrat gemessen werden kann;
c) Messung des Signals des Reportergens; und
d) Bestimmung der Menge an PGN in der Probe mithilfe einer Eichkurve, die eine Beziehung zwischen der Menge an PGN und der Intensität des Signals des Reportergens herstellt, **dadurch gekennzeichnet, dass** besagte Eichkurve, die eine Beziehung zwischen der Menge an PGN und der Intensität des Signals des Reportergens herstellt, entweder mit PAM₃Cys-Ser-(Lys)4-Trihydrochlorid standardisiert oder kalibriert ist oder mit PAM₃Cys-Ser-(Lys)4-Trihydrochlorid erstellt worden ist.

2. Verfahren gemäß Anspruch 1, wobei die Behandlung der Probe mittels Beschallung, Erwärmung und/oder Alkalisierung die Fragmentierung und Desintegration der in der Probe enthaltenen PGN erlaubt, insbesondere diesen die Fähigkeit verleiht, den TLR2-Rezeptor zu aktivieren.

3. Verfahren gemäß Anspruch 1 oder 2, wobei die Behandlung der Probe die Bildung von PGN erlaubt, die überwiegend eine Größe von ungefähr 120 kDa aufweisen.

4. Verfahren gemäß einem der Ansprüche 1 bis 3, wobei das Reportergen eine sezernierte alkalische Phosphatase ist.

5. Verfahren gemäß einem der Ansprüche 1 bis 4, wobei die Zelle eine Zelle der HEK-Blue™ hTLR2-Linie ist.

6. Verfahren gemäß einem der Ansprüche 1 bis 5, wobei die Eichkurve, die eine Beziehung zwischen der Menge an PGN und der Intensität des Signals des Reportergens herstellt, mit PAM₃Cys-Ser-(Lys)4-Trihydrochlorid standardisiert oder kalibriert ist, und wobei die Eichkurve, die eine Beziehung zwischen der Menge an PGN und der Intensität des Signals des Reportergens herstellt, mit den PGN erstellt worden ist, die von einem Bakterium stammen, ausgewählt aus *Staphylococcus aureus, Micrococcus luteus, Bacillus subtilis* und *Alicyclobacillus acidocaldarius,* vorzugsweise ausgewählt aus *Staphylococcus aureus, Micrococcus luteus* und *Alicyclobacillus acidocaldarius.*

7. Verfahren gemäß einem der Ansprüche 1 bis 6, wobei das Verfahren einen vorausgehenden Schritt der Erstellung der Eichkurve unter Verwendung von PGN umfasst, die von einem Bakterium stammen, ausgewählt aus *Staphylococcus aureus, Micrococcus luteus, Bacillus subtilis* und *Alicyclobacillus acidocaldarius.*

8. Verfahren gemäß einem der Ansprüche 1 bis 5, wobei die Eichkurve, die eine Beziehung zwischen der Menge an PGN und der Intensität des Signals des Reportergens herstellt, mit PAM₃Cys-Ser-(Lys)4-Trihydrochlorid erstellt worden ist und wobei das Verfahren einen vorausgehenden Schritt der Erstellung der Eichkurve unter Verwendung von PAM₃Cys-Ser-(Lys)4-Trihydrochlorid umfasst.

9. Verfahren gemäß einem der Ansprüche 1 bis 8, wobei die Probe, falls erforderlich, verdünnt wird, um ein Signal des Reportergens zu generieren, das in dem linearen Bereich der Eichkurve liegt.

10. Verfahren gemäß einem der Ansprüche 1 bis 9, wobei die Probe eine Probe einer Icodextrin-Lösung ist.

11. Kit, das die quantitative Bestimmung von Peptidoglycanen (PGN) in einer Probe von Glucosepolymeren erlaubt, umfassend:
- eine rekombinante Zelle, die einen exogenen TLR2-Rezeptor (Toll-like Receptor 2) und ein Reportergen in direkter Abhängigkeit des TLR2-Rezeptor-assoziierten Signalwegs exprimiert, wobei das Reportergen für ein farbiges oder fluoreszierendes Protein oder ein Protein codiert, dessen Aktivität mit oder ohne Substrat gemessen werden kann; und
- einen PGN-Standard, der vorzugsweise von einem Bakterium stammt, ausgewählt aus *Staphylococcus aureus, Micrococcus luteus, Bacillus subtilis* und *Alicyclobacillus acidocaldarius*;
- gegebenenfalls eine Gebrauchsanleitung, eine Lösung zur Vorbehandlung der Probe;
wobei besagtes Kit außerdem PAM₃Cys-Ser-(Lys)4-Trihydrochlorid umfasst.

12. Verwendung eines Kits für die quantitative Bestimmung von Peptidoglycanen (PGN) in einer Probe von Glucosepolymeren gemäß dem Verfahren nach einem der Ansprüche 1 bis 10, wobei besagtes Kit umfasst:
- eine rekombinante Zelle, die einen exogenen TLR2-Rezeptor (Toll-like Receptor 2) und ein Reportergen in direkter Abhängigkeit des TLR2-Rezeptor-assoziierten Signalwegs exprimiert, wobei das Reportergen für ein farbiges oder fluoreszierendes Protein oder ein Protein codiert, dessen Aktivität mit oder ohne Substrat gemessen werden kann; und
- entweder eine Eichkurve, die eine Beziehung zwischen der Menge an PGN und der Intensität des Signals des Reportergens herstellt, oder einen PGN-Standard, der vorzugsweise von einem Bakterium stammt, ausgewählt aus *Staphylococcus aureus, Micrococcus luteus, Bacillus subtilis und Alicyclobacillus acidocaldarius;*
- gegebenenfalls eine Gebrauchsanleitung, eine Lösung zur Vorbehandlung der Probe;
wobei besagtes Kit außerdem PAM₃Cys-Ser-(Lys)4-Trihydrochlorid umfasst.

## Claims

1. A method of assaying peptidoglycans (PGNs) in a sample of glucose polymer, comprising:
a) treating the sample of glucose polymer by sonication, heating, and/or alkalization;
b) bringing the treated sample or a dilution thereof into contact with a recombinant cell expressing an exogenous TLR2 receptor (Toll-like Receptor 2) and a reporter gene under the direct dependence of the signaling pathway associated with the TLR2 receptor, said reporter gene coding for a colored or fluorescent protein or for a protein whose activity can be measured with or without substrate;
c) measuring the reporter gene signal; and
d) determining the amount of PGN in the sample using a calibration curve of the correspondence between the amount of PGN and the intensity of the reporter gene signal **characterized in that** the calibration curve of the correspondence between the amount of PGN and the intensity of the reporter gene signal is standardized or calibrated with PAM₃Cys-Ser-(Lys)4 trihydrochloride or was prepared with PAM₃Cys-Ser-(Lys)4 trihydrochloride.

2. The method as claimed in claim 1, **characterized in that** treatment of the sample by sonication, heating, and/or alkalization makes it possible to fragment and disintegrate the PGNs contained in the sample, in particular so as to make them capable of activating the TLR2 receptor.

3. The method as claimed in claim 1 or 2, **characterized in that** treatment of the sample makes it possible to generate PGNs predominantly having a size of about 120 kDa.

4. The method as claimed in any one of claims 1-3, **characterized in that** the reporter gene is a secreted alkaline phosphatase.

5. The method as claimed in any one of claims 1-4, **characterized in that** the cell is a cell of the HEK-Blue™ hTLR2 line.

6. The method as claimed in any one of claims 1-5, **characterized in that** the calibration curve of the correspondence between the amount of PGN and the intensity of the reporter gene signal is standardized or calibrated with PAM₃Cys-Ser-(Lys)4 trihydrochloride, and **in that** the calibration curve of the correspondence between the amount of PGN and the intensity of the reporter gene signal was prepared with PGNs derived from a bacterium selected from *Staphylococcus aureus, Micrococcus luteus, Bacillus subtilis* and *Alicyclobacillus acidocaldarius,* preferably from *Staphylococcus aureus, Micrococcus luteus,* and *Alicyclobacillus acidocaldarius.*

7. The method as claimed in any one of claims 1-6, **characterized in that** the method comprises a preliminary step of preparation of the calibration curve using PGNs derived from a bacterium selected from *Staphylococcus aureus, Micrococcus luteus, Bacillus subtilis* and *Alicyclobacillus acidocaldarius.*

8. The method as claimed in any one of claims 1-5, **characterized in that** the calibration curve of the correspondence between the amount of PGN and the intensity of the reporter gene signal was prepared with PAM₃Cys-Ser-(Lys)4 trihydrochloride and **in that** the method comprises a preliminary step of preparation of the calibration curve using PAM₃Cys-Ser-(Lys)4 trihydrochloride.

9. The method as claimed in any one of claims 1-8, **characterized in that** the sample is diluted, if necessary, so as to generate a signal of the reporter gene corresponding to the linear portion of the calibration curve.

10. The method as claimed in any one of claims 1-9, **characterized in that** the sample is a sample of a solution of icodextrin.

11. A kit for assaying peptidoglycans (PGNs) in a sample of glucose polymers comprising:
- a recombinant cell expressing an exogenous TLR2 receptor (Toll-like Receptor 2) and a reporter gene under the direct dependence of the signaling pathway associated with the TLR2 receptor, said reporter gene coding for a colored or fluorescent protein or for a protein whose activity can be measured with or without substrate; and
- a PGN standard, preferably derived from a bacterium selected from *Staphylococcus aureus, Micrococcus luteus, Bacillus subtilis* and *Alicyclobacillus acidocaldarius,* preferably from *Staphylococcus aureus, Micrococcus luteus,* and *Alicyclobacillus acidocaldarius;*
- optionally instructions for use, and a solution for pretreating the sample;.
said kit further comprising PAM₃Cys-Ser-(Lys)4 trihydrochloride.

12. Use of a kit for assaying peptidoglycans (PGNs) in a sample of glucose polymer according to the method as claimed in any one of claims 1-10, said kit comprising:
- a recombinant cell expressing an exogenous TLR2 receptor (Toll-like Receptor 2) and a reporter gene under the direct dependence of the signaling pathway associated with the TLR2 receptor, said reporter gene coding for a colored or fluorescent protein or for a protein whose activity can be measured with or without substrate; and
- a PGN standard, preferably derived from a bacterium selected from *Staphylococcus aureus, Micrococcus luteus, Bacillus subtilis* and *Alicyclobacillus acidocaldarius,* preferably from *Staphylococcus aureus, Micrococcus luteus,* and *Alicyclobacillus acidocaldarius;*
- optionally instructions for use, and a solution for pretreating the sample;.
said kit further comprising PAM₃Cys-Ser-(Lys)4 trihydrochloride.
